# EUROPÄISCHE PATENTSCHRIFT

(11) **EP 1 883 197 B1**
(45) Veröffentlichungstag und Bekanntmachung des Hinweises auf die Patenterteilung: **21.05.2014**
(21) Anmeldenummer: 06117734.1
(22) Anmeldetag: 24.07.2006
(51) Int. Cl.: H04L 29/06, G06F 19/00

(54) **Verfahren zur Herstellung einer Datenverbindung zwischen Medizingeräten und einem Computersystem**
Method for creating a data connection between medical devices and a computer system
Procédé destiné à l'établissement d'une liaison de données entre des appareils médicaux et un système informatique

(43) Veröffentlichungstag der Anmeldung: 30.01.2008
(73) Patentinhaber: Agfa HealthCare, 2640 Mortsel (BE)
(72) Erfinder: Leiteritz, Stefan, 1150 Wien (AT); Schmitt, Jörg, 54317 Osburg (DE)
(74) Vertreter: Linsmeier, Josef

(56) Entgegenhaltungen:
- US-A1- 2006 041 917
- US-A1- 2006 136 594

## Beschreibung

Die Anmeldung betrifft ein Verfahren zur Herstellung einer Datenverbindung zwischen Medizingeräten und einem Computersystem.

In Krankenhäusern werden Softwaresysteme zur Dokumentation von patientenbezogenen Informationen eingesetzt. Bei diesen Informationen handelt es sich unter anderem um Messwerte von Medizingeräten, die den Patientenzustand überwachen und/oder beeinflussen. Die Medizingeräte verfügen im allgemeinen über Schnittstellen, um Daten, wie z.B. gemessene bzw. eingestellte Werte sowie Alarmdaten, elektronisch verfügbar zu machen.

Patientendokumentationssysteme bedienen sich verschiedener Softwaremodule, um die Daten der Medizingeräte zu empfangen und weiterzuverarbeiten, wie z.B. anzuzeigen und/oder zu speichern. Die Medizingeräte sind hierzu an Computern, sog. Interface-PCs, angeschlossen, die die Funktion einer Schnittstelle zwischen den einzelnen Medizingeräten einerseits und den die Daten verarbeitenden Systemen andererseits übernehmen.

Es existiert eine große Anzahl verschiedener Medizingeräte mit unterschiedlichen Kommunikationsprotokollen, die unterschiedliche Schnittstelleneinstellungen erfordern. Herbei handelt es sich beispielsweise um Patientenüberwachungs-, Beatmungs- oder Anästhesiegeräte, wie z.B. *IntelliVue MP90* von Philips Medical Systems, *Solar 8000* von GE Healthcare, *Infinity Delta* von Dräger/Siemens, *AS*/*3* von Datex, *Evita2* von Dräger, *Servo 300* von Maquet, *Zeus* von Dräger bzw. *Aestiva*/*5* von Datex.

Das Anschließen eines neuen Medizingerätes an einen Interface-PC oder der Austausch eines Medizingerätes gegen ein anderes Medizingerät ist im allgemeinen mit einer Reihe von Einstellungen sowohl am Medizingerät als auch am Interface-PC verbunden, die in der Regel vom medizinischen Personal vorgenommen werden müssen.

Zunächst muss die zu einem Medizingerät gehörende Treibersoftware ausgewählt werden. Diese ist für das jeweilige Medizingerät jedoch nicht immer sofort verfügbar oder bekannt. Darüber hinaus kann ein Medizingerät mehrere verschiedene Kommunikationsparameter zulassen. Zu den Kommunikationsparametern zählen z.B. die Datenübertragungsrate (Baudrate), die Anzahl der Datenbits, die Parität, die Anzahl Start- und Stopbits sowie die verwendete Art der Flusssteuerung. Auch sind die zulässigen Kommunikationsparameter nicht immer bekannt, so dass diese erst am Medizingerät abgelesen werden müssen. Dafür müssen gegebenenfalls Systemmenüs geöffnet werden, die durch Passwortabfrage geschützt sein können. Die Eingabe der Kommunikationsparameter wird daher vom betroffenen Personal oft als lästig empfunden oder sogar vergessen und ist außerdem fehleranfällig.

Werden die genannten Einstellungen nicht oder fehlerhaft vorgenommen, so kann keine Datenverbindung zum Medizingerät aufgebaut werden, so dass Daten, wie z.B. mit dem Messgerät gemessene bzw. am Messgerät eingestellte Werte, verloren gehen.

Dokument US 2006/0136594 A1 zeigt den Stand der Technik.

Es ist Aufgabe der Erfindung, ein Verfahren zur Herstellung einer möglichst zuverlässigen Datenverbindung zwischen Medizingeräten und einem Computersystem anzugeben.

Diese Aufgabe wird durch das Verfahren gemäß Anspruch 1 gelöst. Erfindungsgemäß führt das Computersystem, in welchem für jedes der Medizingeräte jeweils mindestens ein Kommunikationsprotokoll zusammen mit den diesem Kommunikationsprotokoll zugeordneten Kommunikationsparametern gespeichert ist, folgende Schritte durch:
a. Setzen von Kommunikationsparametern eines Kommunikationsprotokolls;
b. Empfangen von Daten eines Medizingerätes;
c. Prüfung, ob die vom Medizingerät empfangenen Daten mit Musterdaten, welche für das Kommunikationsprotokoll charakteristisch sind, übereinstimmen;
d. Wiederholung der Schritte a. bis c. mit anderen Kommunikationsparametern des Kommunikationsprotokolls, wenn die vom Medizingerät empfangenen Daten mit den Musterdaten nicht übereinstimmen;
e. Herstellung einer Datenverbindung zwischen dem Medizingerät und dem Computersystem unter Verwendung des Kommunikationsprotokolls, wenn die vom Medizingerät empfangenen Daten mit den Musterdaten übereinstimmen,
   wobei die Schritte a. bis e. mit mindestens einem weiteren Kommunikationsprotokoll wiederholt werden, wenn keine Datenverbindung zwischen dem Medizingerät und dem Computersystem hergestellt werden

Für den Fall, dass das Medizingerät erst auf eine Abfrage hin Daten an das Computersystem sendet, kann zwischen den Schritten a. und b. das Senden einer Abfrage zum Medizingerät vorgesehen sein.

Ein Kommunikationsprotokoll ist eine Vorschrift über die Form, in welcher Daten zwischen den Medizingeräten und dem Computersystem ausgetauscht werden. Diese Vorschrift besteht aus einem Satz von Regeln und Formaten (Syntax).

Unter den Kommunikationsparametern eines Kommunikationsprotokolls sind diejenigen Parameter zu verstehen, deren Werte vor dem Aufbau einer Datenverbindung zwischen dem jeweiligen Medizingerät und dem Computersystem gesetzt werden müssen. Hierzu gehören beispielsweise
- die Datenübertragungsrate (Baudrate), welche die Geschwindigkeit der Datenübertragung in der Einheit Bits/Sekunde angibt,
- die Anzahl der Datenbits in einem Datenwort,
- die Parität, welche zur Fehlererkennung herangezogen wird,
- die Anzahl der Stopbits zur Erkennung des Datenwort-Endes,
- die Anzahl Startbits zur Erkennung des Datenwort-Anfangs und
- die Flusssteuerung (keine, Software oder Hardware).

Die jeweiligen Werte der Kommunikationsparameter sind am Medizingerät eingestellt und müssen zur Herstellung der Datenverbindung in der Software des Computersystems, der sog. Interface-Software, auf dieselben Werte eingestellt werden.

Die bei einem Kommunikationsprotokoll jeweils zu erwartenden Musterdaten können durch Analyse der gemäß Herstellerangaben, z.B. in Form von Beschreibungen der Medizingeräte, zu erwartenden Antwortdaten des Medizingerätes ermittelt werden. Durch die Ermittlung unveränderlicher sowie variabler Abschnitte in den zu erwartenden Antwortdaten kann ein Muster abgeleitet werden, welches dann zur Analyse herangezogen wird. Wird z.B. das Datum vom Gerät übertragen, so kann man dessen Position in der Antwort und gleichzeitig seine Struktur (z.B. "2006-01-31" oder "31.01.2006") leicht ermitteln. Diese Information kann ebenfalls zur Generierung der Musterdaten herangezogen werden. Hierbei werden vorzugsweise reguläre Ausdrücke verwendet. Reguläre Ausdrücke (abgekürzt "RegExp" oder "Regex", englisch "regular expressions") dienen der Beschreibung von (Unter-)Mengen von Zeichenketten mit Hilfe sytaktischer Regeln.

Die erfindungsgemäße Lösung basiert auf dem Gedanken, zwischen dem Computersystem, insbesondere dem sog. Interface-PC, und den daran angeschlossenen einzelnen Medizingeräten automatisch, d.h. ohne zusätzliche Aktionen eines Benutzers, eine Datenverbindung aufzubauen. Hierbei wird das vom jeweiligen Medizingerät verwendete Kommunikationsprotokoll einschließlich der zugehörigen Kommunikationsparameter vom Computersystem automatisch ermittelt und bei der Herstellung der Datenverbindung eingestellt. Eine Erkennung, um welches Medizingerät es sich konkret handelt (z.B. Herstellername und Typenbezeichnung), ist hierbei nicht erforderlich.

Bei der automatischen Ermittlung des passenden Kommunikationsprotokolls wird geprüft, ob ein an der entsprechenden seriellen Schnittstelle des Interface-PCs angeschlossenes Medizingerät ein bestimmtes Kommunikationsprotokoll "spricht". Wenn ja, so wird das dem Kommunikationsprotokoll entsprechende Treibersoftwaremodul des Medizingerätes im Computersystem aktiviert. Als Treibersoftware ist die softwaretechnische Umsetzung des Kommunikationsprotokolls zu verstehen. Die Treibersoftware kommuniziert mit dem Gerät, baut die Datenverbindung auf oder ab, fragt aktuelle Messdaten ab usw.

Wenn hingegen keine passende Antwort empfangen wird, so ist entweder kein Medizingerät mit dem Interface-PC verbunden oder das jeweilige Medizingerät "spricht" dieses Protokoll nicht, sondern ein anderes oder die Kommunikationsparameter stimmen nicht mit denen des Medizingerätes überein. In diesem Fall werden erfindungsgemäß die dem Kommunikationsprotokoll zugeordneten Kommunikationsparameter geändert und die Erkennung erneut versucht.

Sollte danach erneut keine oder eine falsche Antwort empfangen werden, so wird das nächste Softwaremodul aktiviert, welches versucht, unter Heranziehung eines anderen Kommunikationsprotokolls eine Datenverbindung mit dem Medizingerät in der vorstehend beschriebenen Weise herzustellen.

Dieser Vorgang wird mit allen möglichen Softwaremodulen bzw. Kommunikationsprotokollen wiederholt, bis eine Datenverbindung mit dem Medizingerät hergestellt werden kann.

Vorzugsweise läuft das oben beschriebene Verfahren auch dann, wenn kein Medizingerät mit dem Interface-PC verbunden ist. Wird bei dieser Variante der Erfindung ein Medizingerät am Interface-PC angeschlossen, so wird eine Datenverbindung automatisch hergestellt, ohne dass das Verfahren durch eine Benutzeraktion initiiert zu werden braucht.

Die Erfindung wir nachfolgend anhand von Figuren näher erläutert. Es zeigen:
- Fig. 1: ein Computersystem zur Durchführung des erfindungsgemäßen Verfahrens;
- Fig. 2: ein Ablaufdiagramm eines Beispiels des erfindungsgemäßen Verfahrens;
- Fig. 3: ein Ablaufdiagramm eines Teilprozesses des in Fig. 2 dargestellten Verfahrens; und
- Fig. 4: ein Ablaufdiagramm eines weiteren Teilprozesses des in Fig. 2 dargestellten Verfahrens.

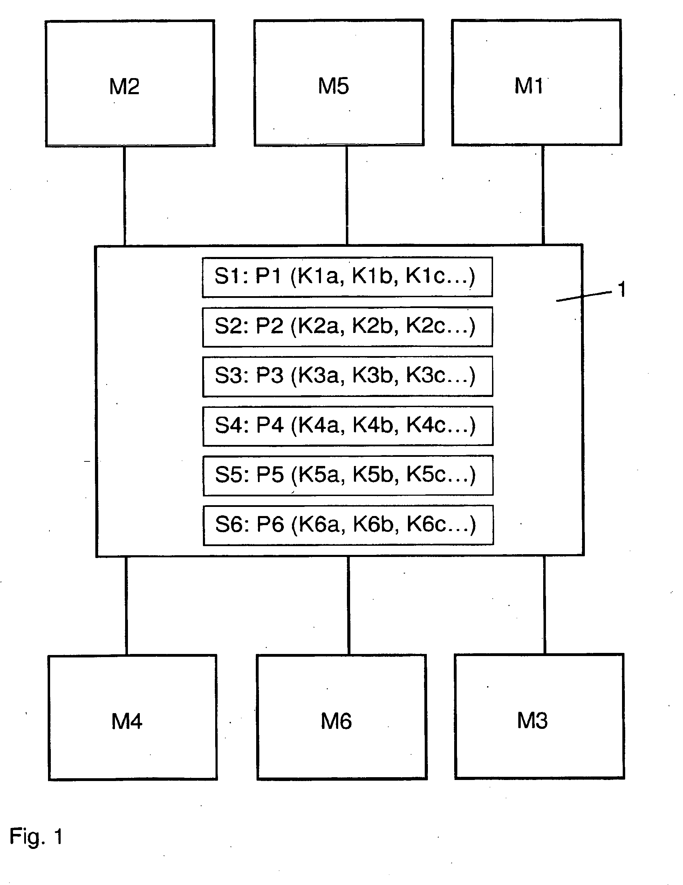

Fig. 1 zeigt ein Computersystem 1 zur Durchführung des erfindungsgemäßen Verfahrens. Mit dem Computersystem 1 sind einzelne Medizingeräte M1 bis M6 durch elektrische Leitungen und/oder drahtlos, z.B. durch Infrarot- oder Radiofrequenzstrahlung, verbunden.

Für jedes der Medizingeräte M1 bis M6 existiert mindestens ein Kommunikationsprotokoll P1 bis P6, dem jeweils mehrere Kommunikationsparameter K1a, K1b, K1c... bis K6a, K6b, K6c... zugeordnet sind.

Die Definition der einzelnen Kommunikationsprotokolle P1 bis P6 einschließlich der jeweils zugeordneten Kommunikationsparameter K1 a, K1 b, K1 c... bis K6a, K6b, K6c... wird in der Regel den Angaben des jeweiligen Medizingeräte-Herstellers entnommen und im Computersystem 1 gespeichert.

Als Beispiele sind nachfolgend die für die Kommunikation mit den Medizingeräten M1 und M2 erforderlichen Kommunikationsprotokolle P1 und P2 und die jeweils zulässigen Werte der Kommunikationsparameter angegeben:

| Medizingerät | M1 | M2 |
|---|---|---|
| Kommunikationsprotokoll | P1 | P2 |
| Gerätetyp | Beatmungsgerät | Anästhesiegerät |
| Baudraten | 2400, 4800, 9600 | 1200, 4800, 19200 |
| Datenbits | 7, 8 | 7, 8 |
| Startbits | 1 | 1 |
| Stopbits | 1 oder 2 | 1 |
| Parität | gerade oder ungerade | gerade |

Zu jedem Kommunikationsprotokoll P1 bis P6 gibt es ein Softwaremodul S1 bis S6, mit dessen Hilfe das zur Kommunikation mit den einzelnen Medizingeräten jeweils erforderliche Kommunikationsprotokoll P1 bis P6 automatisch erkannt werden kann. Die Softwaremodule S1 bis S6 werden vor Beginn des Verfahrens in das Computersystem 1 geladen und dort - wie in Fig. 1 angedeutet - in einer Liste gehalten. Anschließend werden die einzelnen Softwaremodule S1 bis S6 der Reihe nach aktiviert.

Zunächst wird das in der Liste befindliche erste Softwaremodul S1 aktiviert. Dieses versucht, zu einem ersten Medizingerät, z.B. M2, der angeschlossenen Medizingeräte M1 bis M6 entsprechend dem erfindungsgemäßen Verfahren eine Datenverbindung unter Verwendung des ersten Kommunikationsprotokolls P1 aufzubauen.

Kann hierbei keine Datenverbindung zwischen dem ersten Medizingerät M2 und dem Computersystem 1 hergestellt werden, wird das nächste in der Liste befindliche zweite Softwaremodul S2 gestartet. Dieses versucht dann, eine Datenverbindung zum ersten Medizingerät M2 unter Verwendung des zweiten Kommunikationsprotokolls P2 aufzubauen. Ist auch dieser Versuch ohne Erfolg, werden die in der Liste befindlichen weiteren Softwaremodule S3 bis S6 der Reihe nach aktiviert, bis eine Datenverbindung mit dem ersten Medizingerät M2 hergestellt ist.

Das beschriebene Verfahren wird für die anderen Medizingeräte M1, M3 bis M6 entsprechend wiederholt.

Bei dem erfindungsgemäßen Verfahren zur Herstellung einer Datenverbindung unter Verwendung eines Kommunikationsprotokolls, z.B. P1, werden durch das entsprechende Softwaremodul S1 zuerst die Werte der einzelnen Kommunikationsparameter K1a, K1b, K1c... gesetzt, wie z.B. die Datenübertragungsrate, die Anzahl der Datenbits pro Datenwort, die Parität sowie die Anzahl Start- und Stopbits. Vorzugsweise werden dabei die Kommunikationsparameter auf Werte gesetzt, die - z.B. laut Herstellerangaben oder erfahrungsgemäß - bei diesem Kommunikationsprotokoll am häufigsten verwendet werden. In der oben dargestellten Tabelle mit den Kommunikationsprotokollen P1 und P2 sind diese Werte unterstrichen. Im vorliegenden Beispiel des ersten Kommunikationsprotokolls P1 sind dies folgende Werte: Datenübertragungsrate 2400 Bit/s, 8 Datenbits, Anzahl der Start- und Stopbits jeweils 1, Parität gerade.

Es gibt zwei unterschiedliche Typen von Kommunikationsprotokollen. Bei einem ersten Typ muss eine Abfrage zum Medizingerät gesendet werden, bevor das Medizingerät Daten als Antwort zurücksendet. Bei einem zweiten Typ sendet das Medizingerät selbständig Daten.

Werden nun - je nach Typ des Kommunikationsprotokolls gegebenenfalls erst nach Senden einer Abfrage - Daten von dem ersten Medizingerät M2 empfangen, so werden diese Daten mit Musterdaten verglichen, welche für das erste Kommunikationsprotokoll P1 charakteristisch sind und dieses eindeutig von den Musterdaten der anderen Kommunikationsprotokolle P2 bis P6 unterscheidet.

Wenn die Daten des ersten Medizingerätes M2 mit den Musterdaten übereinstimmen, wird eine Datenverbindung zwischen dem ersten Medizingerät M2 und dem Computersystem 1 unter Verwendung des ersten Kommunikationsprotokolls P1 hergestellt. Ist dies nicht der Fall, werden zunächst die Werte der Kommunikationsparameter des ersten Kommunikationsprotokolls P1 geändert und die genannten Schritte auf der Grundlage der geänderten Kommunikationsparameter durchgeführt. Im genannten Beispiel werden die Kommunikationsparameter auf Werte gesetzt, die beim ersten Kommunikationsprotokoll P1 am zweithäufigsten verwendet werden, wie z.B. Datenübertragungsrate 4800 Bit/s, 7 Datenbits pro Datenwort, Anzahl der Startbits 1 und Stopbits 2, Parität ungerade.

Bei der Prüfung der von ersten Medizingerät M2 empfangenen Daten werden darüber hinaus mögliche Kommunikationsfehler, wie z.B. ein sog. Framing Error oder Parity Error, in die Analyse einbezogen und ggf. die Kommunikationsparameter geändert.

Ein Framing Error tritt immer dann auf, wenn die Kommunikationsparameter bei beiden Kommunikationspartnern nicht übereinstimmen, z.B. bei folgenden Einstellungen des Medizingerätes:
7 Datenbits, 1 Startbit, 1 Stopbit, Paritätsbit gerade
   und folgenden Einstellungen des Kommunikationsmoduls am Interface-PC:
8 Datenbits, 1 Startbit, 1 Stopbit, Paritätsbit gerade.

Dies bedeutet, dass beim Senden eines 7-Bit-Datenwortes vom Medizingerät insgesamt 10 Bits verwendet und vom Interface-PC 11 Bits erwartet werden. Der "Rahmen" (das sog. Framing) dieser Kommunikation stimmt also nicht.

Ein Parity Error tritt auf, wenn die Übertragung der Daten gestört wird. Um dies zu erkennen, rechnet der Sender aus den zu übertragenden Bits eine Parität aus und überträgt diese zum Empfänger. Der Empfänger empfängt erst alle Bits, errechnet aus diesen die Parität und vergleicht sie mit der vom Sender übermittelten. Sollte sich der Wert dieses Paritätsbits unterscheiden, so liegt ein Übertragungsfehler vor. Im verwendeten Kommunikationsprotokoll ist dann meist definiert, wie ein solcher Fall zu behandeln ist.

Vorzugsweise wird auch geprüft, ob das Kommunikationsprotokoll zweifelsfrei und eindeutig erkannt wurde. Wenn die erste Antwort zwar mit der erwarteten Antwort übereinstimmt, kann es allerdings noch der Fall sein, dass auch ein anderes Kommunikationsprotokoll die passende Antwort erzeugt hätte. In einem solchen Fall sind weitere Abfragen notwendig, bis das verwendete Kommunikationsprotokoll zweifelsfrei identifiziert ist.

Das folgende Beispiel zweier ähnlicher Kommunikationsprotokolle soll dies verdeutlichen: Bei beiden Kommunikationsprotokollen wird die Kommunikation durch ein entsprechendes Kommando aufgebaut (Initialize Communication Command). Beim ersten Protokoll bedeutet der Kommando-Code 0x2D die Abfrage nach den aktuellen oberen Alarmgrenzen. Beim zweiten Protokoll bedeutet das gleiche Kommando aber die Abfrage der aktuellen Monitordaten. Der Kommando-Code 0x52 bedeutet beim ersten Protokoll die Abfrage der Geräteidentifikation. Um die Geräteidentifikation im zweiten Protokoll abzufragen, ist aber der Kommando-Code 0x2C zu verwenden.

Hieraus ergibt sich, dass allein der Vergleich der Antwort des ICC-Kommandos nicht ausreicht, um das Protokoll eindeutig zu identifizieren, sondern dass noch weitere Abfragen und Vergleiche (z.B. die Abfrage der Geräte-Identifikation) notwendig sind.

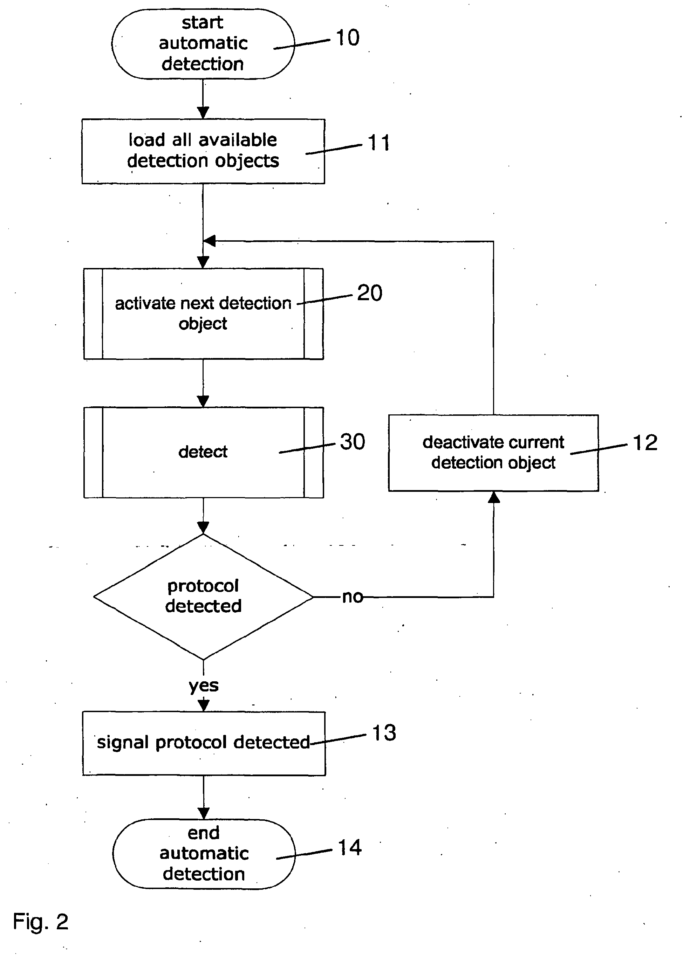

Fig. 2 zeigt ein Ablaufdiagramm eines Beispiels des erfindungsgemäßen Verfahrens zur automatischen Geräteerkennung.

Nach dem Start 10 des Verfahrens werden zunächst alle zur Erkennung der unterschiedlichen Kommunikationsprotokolle vorhandenen Softwaremodule (detection objects) geladen 11 und in einer Liste angeordnet.

In einem weiteren Teilprozess 20 des Verfahrens wird dann das erste bzw. jeweils nächste Softwaremodul zur Erkennung eines Kommunikationsprotokolls gestartet.

In dem darauf folgenden Teilprozess 30 werden dann verschiedene Schritte zur automatischen Erkennung des Kommunikationsprotokolls durchgeführt. Falls das Kommunikationsprotokoll nicht mit dem erforderlichen Protokoll des Medizingerätes übereinstimmt, wird bzw. werden das erste Softwaremodul deaktiviert 12 und die Teilprozesse 20 und 30 für weitere in der Liste angeordneten Softwaremodule zur Erkennung der weiteren Kommunikationsprotokolle solange wiederholt, bis das für das jeweilige Medizingerät passende Kommunikationsprotokoll eindeutig identifiziert worden ist 13. In diesem Fall wird eine Datenverbindung zwischen dem Medizingerät und dem Computersystem unter Verwendung des ermittelten Kommunikationsprotokolls hergestellt und das Verfahren zur automatischen Geräteerkennung beendet 14.

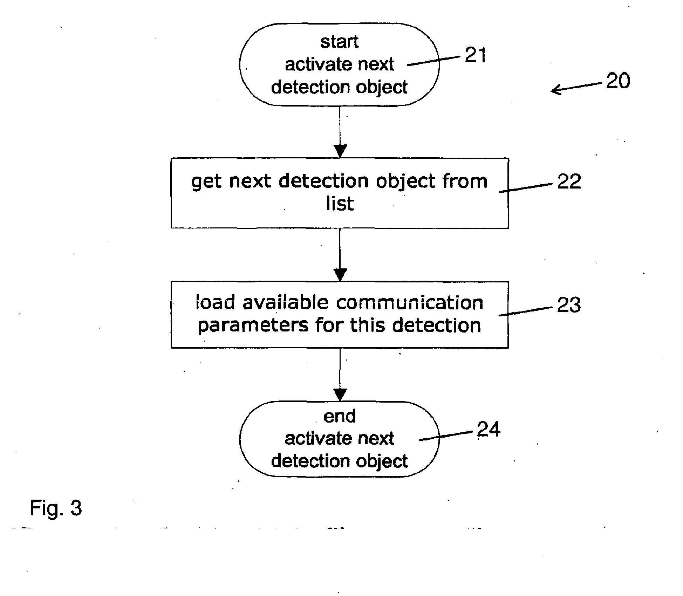

Fig. 3 zeigt ein Ablaufdiagramm des Teilprozesses 20 zum Starten des ersten Softwaremoduls bzw. weiterer Softwaremodule zur Erkennung von Kommunikationsprotokollen.

Nach dem Start 21 dieses Teilprozesses 20 wird das in der Liste jeweils als nächstes angeordnete Softwaremodul herangezogen 22. Anschließend werden die diesem Kommunikationsprotokoll zugeordneten Kommunikationsparameter und deren - vorzugsweise am wahrscheinlichsten verwendeten - Werte geladen bzw. gesetzt 23. Der Teilprozess 20 wird anschließend beendet 24.

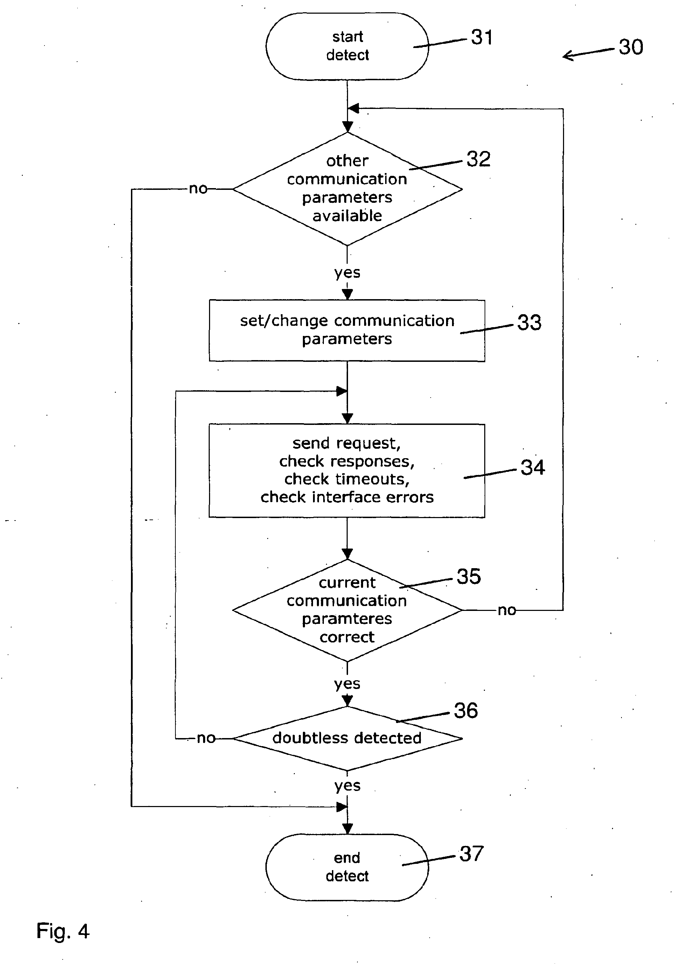

Der sich an den Teilprozess 20 anschließende Teilprozess 30 ist im Detail in Fig. 4 dargestellt.

Nach dem Start 31 wird zunächst überprüft, ob Kommunikationsparameter bzw. weitere Kommunikationsparameter vorliegen 32. Ist dies nicht der Fall, so wird der Teilprozess 30 beendet 37. Für den Fall, dass (weitere) Kommunikationsparameter vorliegen, werden diese eingestellt bzw. verändert 33. Daraufhin wird an das Medizingerät eine Abfrage gesendet und die vom Medizingerät erhaltenen Antworten überprüft. Je nach Typ des Kommunikationsprotokolls kann das Senden einer Abfrage ggf. entfallen. Sollte die nachfolgende Prüfung 35 ergeben, dass die Kommunikationsparameter nicht mit denen des jeweiligen Medizingerätes übereinstimmen, werden die oben genannten Schritte 32 bis 34 für weitere Kommunikationsparameter, sofern solche vorliegen, wiederholt.

Für den Fall, dass die eingestellten Kommunikationsparameter mit denen des Medizingerätes übereinstimmen, wird in einem weiteren Verfahrensschritt 36 überprüft, ob das jeweilige Kommunikationsprotokoll eindeutig und zweifelsfrei erkannt worden ist. Ist dies nicht der Fall, werden die Schritte 34 und 35 wiederholt, indem weitere Antworten des Medizingerätes überprüft werden. Ist eine zweifelsfreie Erkennung des jeweiligen Kommunikationsprotokolls gegeben, kann der Teilprozess 30 beendet werden 37.

## Patentansprüche

1. Verfahren zur Herstellung einer Datenverbindung zwischen Medizingeräten (M1 - M6) und einem Computersystem (1), wobei in dem Computersystem (1) für jedes der Medizingeräte (M1 - M6) mindestens ein Kommunikationsprotokoll (P1 - P6) zusammen mit den diesem Kommunikationsprotokoll (P1 - P6) zugeordneten Kommunikationsparametern (K1a, K1b, K1c...-K6a, K6b. K6c...) gespeichert ist, mit folgenden Schritten, welche von dem Computersystem (1) durchgeführt werden:
a. Setzen von Kommunikationsparametern (K1a, K1b, K1c... - K6a, K6b, K6c...) eines Kommunikationsprotokolls (P1 - P6):
b. Empfangen von Daten eines Medizingerätes (M1 - M6);
c. Prüfung, ob die vom Medizingerät (M1 - M6) empfangenen Daten mit Musterdaten, welche für das Kommunikationsprotokoll (P1 - P6) charakteristisch sind, übereinstimmen;
d. Wiederholung der Schritte a. bis c. mit anderen Kommunikationsparametern (K1a, K1b, K1c... - K6a, K6b, K6c...) des Kommunikationsprotokolls (P1 - P6), wenn die vom Medizingerät (M1 - M6) empfangenen Daten mit den Musterdaten nicht übereinstimmen;
e. Herstellung einer Datenverbindung zwischen dem Medizingerät (M1 - M6) und dem Computersystem (1) unter Verwendung des Kommunikationsprotokolls (P1 - P6), wenn die vom Medizingerät (M1 - M6) empfangenen Daten mit den Musterdaten übereinstimmen,
wobei die Schritte a. bis e. mit mindestens einem weiteren Kommunikationsprotokoll (P1 - P6) wiederholt werden, wenn keine Datenverbindung zwischen dem Medizingerät (M1 - M6) und dem Computersystem (1) hergestellt werden konnte.

2. Verfahren nach Anspruch 1, wobei die Schritte a. bis e. für mindestens ein weiteres Medizingerät (M1 - M6) wiederholt werden.

3. Verfahren nach einem der vorausgehenden Ansprüche, wobei die einem Kommunikationsprotokoll (P1 - P6) zugeordneten Kommunikationsparameter (K1a, K1b, K1c... - K6a, K6b, K6c...) in diesem Kommunikationsprotokoll (P1 - P6) mit unterschiedlicher Wahrscheinlichkeit vorkommen können und in Schritt a. diejenigen Kommunikationsparameter (K1a, K1b, K1c... - K6a, K6b, K6c...) gesetzt werden, welche die höchste Wahrscheinlichkeit aufweise.

4. Verfahren nach einem der vorausgehenden Ansprüche, wobei sich die für das jeweilige Kommunikationsprotokoll (P1 - P6) charakteristischen Musterdaten von Musterdaten anderer Kommunikationsprotokolle (P1 - P6) eindeutig unterscheiden.

5. Verfahren nach einem der vorausgehenden Ansprüche, wobei die für ein Kommunikationsprotokoll (P1 - P6) charakteristischen Musterdaten durch eine Analyse von Antwortdaten eines Medizingerätes (M1 - M6) ermittelt werden.

6. Verfahren nach einem der vorausgehenden Ansprüche, wobei vor Schritt b. eine Abfrage zum Medizingerät (M1 - M6) gesendet wird und in Schritt b. die auf die Abfrage vom Medizingerät (M1 - M6) gesendeten Daten des Medizingerätes (M1 - M6) empfangen werden.

7. Verfahren nach einem der vorausgehenden Ansprüche, wobei vor der Herstellung der Datenverbindung in Schritt e. überprüft wird, ob eine eindeutige Identifizierung des ersten Kommunikationsprotokolls (P1 - P6) gegeben ist.

8. Verfahren nach einem der vorausgehenden Ansprüche, wobei zur Herstellung der Datenverbindung in Schritt e. ein dem verwendeten Kommunikationsprotokoll (P1 - P6) zugeordnetes Treibersoftwaremodul des Medizingerätes (M1 - M6) im Computersystem (1) aktiviert wird.

9. Verfahren nach einem der vorausgehenden Ansprüche, welches auch dann ausgeführt wird, wenn kein Medizingerät (M1 - M6) mit dem Computersystem (1) verbunden ist.

10. Computersystem zur Durchführung des Verfahrens nach einem der voransgehenden Ansprüche.

## Claims

1. A method for creating a data connection between medical appliances (M1 - M6) and a computer system (1), whereby in this computer system (1) for each of the medical appliances (M1 - M6) at least one communication protocol (P1 - P6) is stored together with the communication parameters (K1a, K1b, K1c... - K6a, K6b, K6c...) assigned to this communication protocol (P1 - P6), said method comprising the following steps which are carried out by the computer system (1) :
a. setting communication parameters (K1a, K1b, K1c... - K6a, K6b, K6c...) of a communication protocol (P1 - P6);
b. receiving data from a medical appliance (M1 - M6);
c. checking whether the data received from the medical appliance (M1 - M6) correspond to sample data which are characteristic for the communication protocol (P1 - P6);
d. repeating steps a. to c. with other communication parameters (K1a, K1b, K1c... - K6a, K6b, K6c...) of the communication protocol (P1 - P6) if the data received from the medical appliance (M1 - M6) do not correspond to the sample data;
e. creating a data connection between the medical appliance (M1 - M6) and the computer system (1) using the communication protocol (P1 - P6) if the data received from the medical appliance (M1 - M6) correspond to the sample data;
the steps a. to e. being repeated with at least one further communication protocol (P1 - P6) if it was not possible to create a data connection between the medical appliance (M1 - M6) and the computer system (1).

2. The method according to claim 1, wherein the steps a. to e. are repeated for at least one further medical appliance (M1 - M6).

3. The method according to any of the preceding claims, wherein the communication parameters (K1a, K1b, K1c... - K6a, K6b, K6c...) which are assigned to a communication protocol (P1 - P6) can occur in this communication protocol (P1 - P6) with different likelihood and in step a. those communication parameters (K1a, K1b, K1c... - K6a, K6b, K6c...) are set which have the highest likelihood.

4. The method according to any of the preceding claims, wherein the sample data that are characteristic for the respective communication protocol (P1 - P6) unambiguously differ from sample data of other communication protocols (P1 - P6).

5. The method according to any of the preceding claims, wherein the sample data that are characteristic for a communication protocol (P1 - P6) are determined by an analysis of response data from a medical appliance (M1 - M6).

6. The method according to any of the preceding claims, wherein before step b. a query is sent to the medical appliance (M1 - M6) and in step b. the data of the medical appliance (M1 - M6) are received which are sent by the medical appliance (M1 - M6) upon the query made to it.

7. The method according to any of the preceding claims, wherein before creating the data connection in step e., it is examined whether the first communication protocol (P1 - P6) has been unambiguously identified.

8. The method according to any of the preceding claims, wherein a driver software module of the medical appliance (M1 - M6) assigned to the used communication protocol (P1 - P6) is activated in the computer system (1) in order to create the data connection in step e.

9. The method according to any of the preceding claims that is also implemented if no medical appliance (M1 - M6) is connected to the computer system (1).

10. Computer system for implementing the method according to any of the preceding claims.

## Revendications

1. Procédé destiné à l'établissement d'une liaison de données entre des appareils médicaux (M1 - M6) et un système informatique (1), au moins un protocole de communication (P1 - P6), ainsi que les paramètres de communication (K1a, K1b, K1c... - K6a, K6b, K6c...) assignés à ce protocole de communication (P1 - P6), étant mémorisé dans ledit système informatique (1) pour chacun des appareils médicaux (M1 - M6), ledit procédé comprenant les étapes suivantes exécutées par le système informatique (1) et consistant à :
a. établir des paramètres de communication (K1a, K1b, K1c... - K6a, K6b, K6c...) d'un protocole de communication (P1 - P6);
b. recevoir des données d'un appareil médical (M1 - M6);
c. vérifier si les données reçues de l'appareil médical (M1 - M6) correspondent à des données d'exemple qui sont caractéristiques du protocole de communication (P1 - P6);
d. répéter les étapes a. à c. avec d'autres paramètres de communication (K1a, K1b, K1c... - K6a, K6b, K6c...) du protocole de communication (P1 - P6) si les données reçues de l'appareil médical (M1 - M6) ne correspondent pas aux données d'exemple;
e. établir une liaison de données entre l'appareil médical (M1 - M6) et le système informatique (1) en utilisant le protocole de communication (P1 - P6) si les données reçues de l'appareil médical (M1 - M6) correspondent aux données d'exemple;
les étapes a. à e. étant répétées avec au moins un autre protocole de communication (P1 - P6) s'il n'était pas possible d'établir une liaison de données entre l'appareil médical (M1 - M6) et le système informatique (1).

2. Procédé selon la revendication 1, **caractérisé en ce que** les étapes a. à e. sont répétées pour au moins un autre appareil médical (M1 - M6).

3. Procédé selon l'une quelconque des revendications précédentes, **caractérisé en ce que** les paramètres de communication (K1a, K1b, K1c... - K6a, K6b, K6c...) assignés à un protocole de communication (P1 - P6) peuvent se présenter avec une probabilité différente dans ce protocole de communication (P1 - P6) et que l'étape a. consiste à établir les paramètres de communication (K1a, K1b, K1c... - K6a, K6b, K6c...) qui présentent la probabilité la plus élevée.

4. Procédé selon l'une quelconque des revendications précédentes, **caractérisé en ce que** les données d'exemple qui sont caractéristiques du protocole de communication (P1 - P6) respectif diffèrent de manière univoque de données d'exemple d'autres protocoles de communication (P1 - P6).

5. Procédé selon l'une quelconque des revendications précédentes, **caractérisé en ce que** les données d'exemple qui sont caractéristiques d'un protocole de communication (P1 - P6) sont déterminées par une analyse de données de réponse d'un appareil médical (M1 - M6).

6. Procédé selon l'une quelconque des revendications précédentes, **caractérisé en ce qu'**avant l'étape b., une demande est transmise à l'appareil médical (M1 - M6) et que l'étape b. consiste à recevoir les données de l'appareil médical (M1 - M6) qui sont transmises par l'appareil médical (M1 - M6) suite à la demande transmise à elle.

7. Procédé selon l'une quelconque des revendications précédentes, **caractérisé en ce qu'**avant d'établir la liaison de données dans l'étape e., il est vérifié si le premier protocole de communication (P1 - P6) a été identifié de manière univoque.

8. Procédé selon l'une quelconque des revendications précédentes, **caractérisé en ce qu'**un module de logiciel de pilote de l'appareil médical (M1 - M6) assigné au protocole de communication (P1 - P6) utilisé est activé dans le système informatique (1) afin d'établir la liaison de données dans l'étape e.

9. Procédé selon l'une quelconque des revendications précédentes qui est également implémenté si aucun appareil médical (M1 - M6) n'est connecté au système informatique (1).

10. Système informatique destiné à mettre en oeuvre le procédé selon l'une quelconque des revendications précédentes.
